# EUROPEAN PATENT APPLICATION

(11) **EP 4 332 271 A1**
(43) Date of publication of application: **06.03.2024**
(21) Application number: 21963805.3
(22) Date of filing: 29.11.2021
(51) Int. Cl.: C25B 1/044, C01B 3/04

(54) **EFFICIENT OXYHYDROGEN GENERATION DEVICE FOR MEDICAL CARE, AND METHOD THEREFOR**

(30) Priority: 12.11.2021 CN 202111341489; 12.11.2021 CN 202122773182 U
(71) Applicant: Dalian Institute of Chemical Physics, Chinese Academy of Sciences, Dalian, Liaoning 116023 (CN)
(72) Inventor: DENG, Dehui, Dalian, Liaoning 116023 (CN); LIU, Yanting, Dalian, Liaoning 116023 (CN); BO, Xin, Dalian, Liaoning 116023 (CN); SHU, Yunmao, Dalian, Liaoning 116023 (CN)
(74) Representative: Proi World Intellectual Property GmbH
(86) International application number: PCT/CN2021/133946
(87) International publication number: WO 2023/082363

(57) **Abstract**

The present invention provides an efficient oxyhydrogen generation device for medical care, mainly comprising a housing, an upper cover and a bottom cover which form a main frame, where the housing is composed of a front part and a rear part, the bottom cover is fastened at the bottom of the housing to form a space for accommodating an electrolytic cell, a water supply tank, a water supply tank upper cover and a secondary water tank; the upper cover is fastened on the upper part of the housing and is provided with an atomized gas circulation part, a supply part for supplying water to the water supply tank, and a control panel for controlling the operation of an electrolyzing water hydrogen-oxygen generator; and oxyhydrogen generated by electrolysis of the electrolytic cell sequentially enters the water supply tank and the secondary water tank by means of a gas guide plate and is cleaned, and the cleaned gas is discharged from a gas circulation part.

## Description

### TECHNICAL FIELD

The present invention relates to the technical field of oxyhydrogen preparation, particular to a high-efficiency oxyhydrogen generation device for medical care and a using method thereof.

### BACKGROUND ART

In recent years, hydrogen has been widely applied to the fields of medical treatment, health care and health preservation, and people have paid increasing attention to hydrogen-containing gas/liquid products. By utilizing the biological effects of hydrogen, such as anti-oxidation, anti-inflammation, anti-apoptosis and cell repair, the effect of improving the physical condition and preventing and treating diseases can be achieved. Many research results show that hydrogen can selectively scavenge malignant free radicals in the body, has health effects such as immune regulation and metabolic regulation, and can be used for auxiliary treatment of cerebral ischemia, senile dementia, lung injury, and kidney injury, as well as for tumor treatment and sleep improvement. In addition, hydrogen has cosmetic effects such as beauty and anti-aging, anti-ultraviolet damage, skin tissue repair, etc.

At present, hydrogen-containing products on the market include canned hydrogen water, hydrogen machines and hydrogen water cups that can generate hydrogen, and hydrogen-oxygen generators. Currently, canned hydrogen water has several disadvantages including the low hydrogen content, possible hydrogen loss during transportation or storage, and gradual decrease of hydrogen concentration over time. Hydrogen-rich water prepared by the hydrogen water cup possibly contains electrode catalyst corrosion products and membrane decomposition products. Hydrogen machines or hydrogen generators in medical institutions have defects such as complex structure, high power, and high energy consumption. Therefore, it is necessary to provide a novel high-efficiency oxyhydrogen generation device for medical care to solve the deficiencies in the prior art.

### SUMMARY

To solve the technical problems of the existing hydrogen production equipment including the complex structure, high power, and weak safety, the present invention provides a high-efficiency oxyhydrogen generating device for medical care and a using method thereof. According to the present invention, oxyhydrogen is directly produced based on the principle of electrolyzing water. The oxyhydrogen produced in an electrolytic cell is cleaned and filtered once through a water supply tank, and the cleaned gas is then directly discharged or discharged through an atomizer after secondary cleaning through a secondary water tank. At the same time, the water supply tank is used to supply water to the electrolytic cell on time to ensure that the water level does not exceed the safety threshold, so that the oxyhydrogen can be produced immediately for use, having the advantages of high efficiency, controllable flow, good safety, portability, and mobility, the oxyhydrogen is suitable for use in hospitals and homes.

The technical means employed by the present invention are as follows:
A high-efficiency oxyhydrogen generating device for medical care includes:
A housing configured to hold the electrolytic cell, the water supply tank, and the secondary water tank;
An upper cover fastened to an upper part of the housing and provided with a gas circulation part, a supply part for supplying water to the water supply tank and a control panel for controlling the operation of an electrolyzing water hydrogen-oxygen generator;
A bottom cover fastened to an bottom of the housing.

The water supply tank is provided with a water supply tank upper cover, and an accommodating space is disposed inside the water supply tank upper cover. The upper cover is sleeved on the outer side of the water supply tank upper cover and is fastened to the housing. An air guide plate, a circuit board, an atomizer, and a fixing plate for fixing the circuit board are disposed in the accommodating space.

The electrolytic cell is disposed on the bottom cover at the bottom of the housing, and is connected to the water supply tank thereabove through a pipeline. The oxyhydrogen generated by the electrolysis of the electrolytic cell passes through a gas outlet pipe and the air guide plate in the water supply tank upper cover to enter the water supply tank for primary cleaning, and then enters the secondary water tank through the air guide plate for secondary cleaning, and the cleaned gas is discharged from the gas circulation part.

Further, the electrolytic cell is an electrolytic cell with heat dissipation fins. The electrolytic cell includes an electrolytic tank, and an electrolytic stack and an electrolyte placed inside the electrolytic tank. The electrodes in the electrolytic stack are disposed in a serial-parallel connection mode.

Further, the electrolytic tank is provided with a gas outlet pipe, a pressure sensor, a metal probe insertion port of a liquid level sensor, a safety valve, and a water injection port connected to a pump. The water injection port is connected to the water supply tank through a pipeline.

Further, the heat dissipation fins are disposed around the electrolytic tank. Heat dissipation openings for heat dissipation are disposed on both sides of the housing, and a fan assembly is disposed inside the housing corresponding to each the heat dissipation opening.

Further, two gas guide grooves are disposed on the air guide plate. A gas outlet and a gas inlet are disposed at both ends of each the gas guide grooves, and a gas guide groove sealing cover is disposed on each the gas guide groove.

Further, each of the water supply tank and the secondary water tank is further provided with a device for generating fine bubbles.

Further, the secondary water tank is installed on one side of the housing through a secondary water tank base, and the secondary water tank is further used as an observation window for observing the gas washing situation therein.

Further, a cooling plate is further disposed between the water storage tank and the gas guide plate.

Further, the atomizer is fixed on the fixing plate. The gas after secondary washing can be discharged from the gas circulation part after optionally passing through the atomizer.

Further, the fan assembly includes a fan, a fan fixing bracket, and a wind direction guide groove disposed at the front end of the fan.

Further, the lower end of the bottom cover is provided with casters, and the housing is further provided with a pull rod.

The present invention further discloses a method for using the above high-efficiency oxyhydrogen generation device for medical care. The oxyhydrogen generation device is started after adding an appropriate amount of water. Oxyhydrogen generated by the electrolytic cell is discharged into an gas guide channel on the air guide plate through the gas outlet pipe and enters the water supply tank for primary cleaning, and then enters the secondary water tank through the gas guide plate for secondary cleaning. The cleaned gas is directly discharged from the gas circulation part or discharged after passing through the atomizer.

Compared with the prior art, the present invention has the following advantages:
1. The electrolytic cell provided by the present invention is an electrolytic cell with heat dissipation fins. The heat dissipation fins are disposed on the surface of the electrolytic cell to realize the heat dissipation effect, and in addition, the fan assembly is configured to further dissipate heat for the device, which can greatly reduce the working temperature of the electrolytic cell, and is environment-friendly and energy-saving.
2. The electrolytic stack provided by the present invention, by sharing a negative electrode and arranging the electrodes in a combination of serial and parallel connection, has a lower stack input voltage, and can achieve high gas output at a safe voltage (<36V), with relatively low power consumption and safer use.
3. The gas guide plate provided by the present invention, through the gas guide groove arranged thereon, introduces the generated gas into the water supply tank in order for primary washing on demand, then the gas is introduced into the secondary water tank for secondary washing, and finally the gas can be optionally introduced into the atomizer for atomization or directly discharged. After washing twice, the oxyhydrogen at the outlet is purer.
4. The secondary water tank provided by the present invention can be further used as an observation window to observe the internal gas production and washing situation while performing secondary washing of the gas, and controls the gas production in real time through the control panel.
5. The water supply tank in the present invention is connected to the electrolytic cell through a pipeline. When the water level detector detects that the water level in the electrolytic cell is insufficient, the water supply tank will supply water in real time to ensure the smooth operation of the device.

To sum up, the application of the technical solution of the present invention can realize the primary/secondary cleaning and filtering of the oxyhydrogen generated by the electrolytic cell in real time, and adjust the gas production and supply of atomized gas according to the user's needs. The present invention has the advantages such as structural optimization, compact arrangement, clean and controllable gas production, and convenient use.

Based on the above reasons, the present invention can be widely promoted in oxyhydrogen preparation and other fields.

### BRIEF DESCRIPTION OF THE DRAWINGS

In order to more clearly describe the technical solutions in the examples of the present invention or in the prior art, a brief introduction to the accompanying drawings required for the description of the examples or the prior art will be made below. Apparently, the accompanying drawings in the following description are merely some embodiments of the present invention, and those of ordinary skill in the art would also be able to derive other drawings from these drawings without making creative efforts.
FIG. 1 is an exploded view of the structure of a high-efficiency oxyhydrogen generation device for medical care of the present invention.
FIG. 2 is an exploded view of the structure of an electrolytic cell with heat dissipation fins of the present invention.
FIG. 3 is an exploded view of the structure of a fan assembly of the present invention.
FIG. 4 is a schematic diagram of the structure of a gas guide plate of the present invention.
FIG. 5 is a partial sectional view of the high-efficiency oxyhydrogen generation device for medical care of the present invention.
FIG. 6 is a sectional view of the structure of a secondary water tank of the present invention.
FIG. 7 is a schematic diagram of the structure of an electric stack in a combination of serial and parallel connection according to the present invention.

In the figures: 1. upper cover; 2. atomization port; 3. atomization cover; 4. control panel; 5. power button; 6. water injection port; 7. sealing cover of water injection port; 8. side plate sealing element; 9. circuit board; 10. atomization chamber; 11. fixing plate; 12. atomizer; 13. gas guide plate; 14. second gas guide groove; 15. first gas guide groove; 16. sealing cover of gas guide groove; 17. cooling plate; 18. water supply tank upper cover; 19. metal probe of water level detector; 20. housing; 21. pull rod; 22. heat dissipation opening; 23. water supply tank; 24. fan assembly; 25. secondary water tank base; 26. secondary water tank; 27. electrolytic cell with heat dissipation fins; 28. gas outlet pipe; 29. pressure sensor; 30. metal probe insertion port; 31. fixing bracket; 32. safety valve; 33. bottom cover; 34. casters; 35. electrolytic tank; 36. electrolytic stack; 37. heat dissipation fins; 38. upper cover of electrolytic tank; 39. liquid injection port of electrolytic cell; 40. fan fixing bracket; 41. fan; 42. wind direction guide groove; 43. first gas outlet; 44. first gas inlet; 45. third gas outlet; 46. third gas inlet; 47. sixth gas outlet; 48. water injection channel; 49. second gas inlet; 50. second gas outlet; 51. drainage opening of electrolytic cell; 52. drainage opening of water supply tank; 53. fourth gas inlet; 54. fourth gas outlet; 55. fifth gas outlet.

### DETAILED DESCRIPTION OF THE EMBODIMENTS

It should be noted that, in the case of no conflicts, the embodiments and the features in the embodiments of the present invention can be combined mutually. The present invention will be described in detail below with reference to the accompanying drawings and the embodiments.

To make the objectives, technical solutions and advantages of embodiments of the present disclosure more obvious, the technical solutions of the present disclosure will be clearly and completely described below in conjunction with the accompanying drawings in the embodiments of the present disclosure, and obviously, the described embodiments are some, rather than all of the embodiments of the present disclosure. The following description of at least one example embodiment is merely illustrative in nature, and is in no way intended to limit the present disclosure, an application or use thereof. Based on the embodiments of the present disclosure, all other embodiments acquired by those ordinary skilled in the art without making creative efforts fall within the scope of protection of the present disclosure.

It should be noted that the terms used herein are only intended to describe specific embodiments and are not intended to limit the example embodiments of the present disclosure. As used herein, unless indicated obviously in the context, a singular form is also intended to include a plural form. In addition, it should also be understood that the terms "include" and/or "comprise" used in this specification indicate features, steps, operations, devices, components and/or their combinations.

Except as otherwise specifically set forth, the relative arrangement of components and steps, numerical expressions and numerical values set forth in these embodiments do not limit the scope of the present invention. In addition, it should be clear that, for ease of description, sizes of the various components shown in the accompanying drawings are not drawn according to actual proportional relationships. Technologies, methods, and devices known to those of ordinary skill in the relevant fields may not be discussed in detail, but where appropriate, the technologies, methods, and devices should be considered as a part of the authorization specification. In all the examples shown and discussed herein, any specific value should be interpreted as merely example rather than limiting. Therefore, other examples of the example embodiments may have different values. It should be noted that similar reference signs and letters represent similar items in the accompanying drawings below. Therefore, once an item is defined in one accompanying drawing, the item does not need to be further discussed in a subsequent accompanying drawing.

In the description of the present invention, it should be noted that orientations or position relationships indicated by orientation terms "front, rear, upper, lower, left, and right", "transverse, vertical, perpendicular, and horizontal", "top and bottom", and the like are usually based on orientations or position relationships shown in the accompanying drawings, and these terms are only used to facilitate description of the present invention and simplification of the description. In the absence of description to the contrary, these orientation terms do not indicate or imply that the apparatus or element referred to must have a specific orientation or be constructed and operated in a specific orientation, and therefore cannot be understood as a limitation on the protection scope of the present invention: orientation words "inner and outer" refer to the inside and outside relative to the contour of each component.

For ease of description, spatially relative terms such as "on", "over", "on the upper surface", and "above" can be used here, to describe a spatial positional relationship between one device or feature and another device or feature shown in the figures. It should be understood that the spatially relative terms are intended to include different orientations in use or operation other than the orientation of the device described in the figure. For example, if the device in the figure is inverted, the device described as "above another device or structure" or "on another device or structure" is then be positioned as being "below another device or structure" or "beneath a device or structure". Therefore, the exemplary term "above" can include both orientations "above" and "below". The device can also be positioned in other different ways (rotating by 90 degrees or in another orientation), and the spatially relative description used herein is explained accordingly.

In addition, it should be noted that using terms such as "first-stage" and "second-stage" to define components is only for the convenience of distinguishing the corresponding components. Unless otherwise stated, the foregoing words have no special meaning and therefore cannot be understood as a limitation on the protection scope of the present invention.

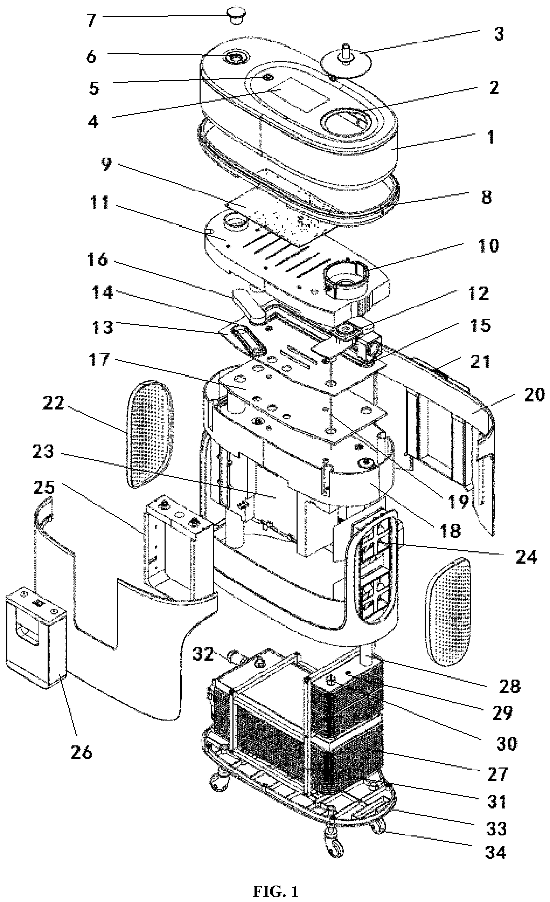

As shown in FIG. 1, the present invention provides a high-efficiency oxyhydrogen generation device for medical care, where the main frame thereof mainly includes a housing 20, an upper cover 1, and a bottom cover 33. The housing 20 is composed of front and rear parts and the bottom cover 33 is fastened at the bottom of the housing to form a space for accommodating an electrolytic cell, a water supply tank 23, a water supply tank upper cover 18, and a secondary water tank 26. The upper cover 1 fastened to the upper part of the housing, and is provided with an atomized gas circulation part, a supply part for supplying water to the water supply tank, and a control panel 4 for controlling the operation of an electrolyzing water hydrogen-oxygen generator.

The water supply tank 23 is provided with the water supply tank upper cover 18, an accommodating space is disposed inside the water supply tank upper cover 18, and the upper cover 1 is sleeved on the outer side of the water supply tank upper cover 18 and is fastened to the housing 20. An air guide plate 13, a circuit board 9 connected to the control panel 4, an atomizer 12, and a fixing plate 11 for fixing the circuit board 9, and the atomizer 12 are disposed in the accommodating space. The atomized gas circulation part is mainly composed of the atomizer 12 disposed in an atomization chamber 10 on the fixing plate 11 and an atomization cover 3 on the upper cover 1. The atomized gas is discharged from an atomization port 2 arranged on the upper cover 1. Atomization or not depends on actual needs. A power button 5 on the upper cover 1 is configured to control the start of the control panel 4, and also the operation of the entire device. The control panel 4 is capable to control the adjustment over the gas production of the entire device, to control the water supply tank 23 to supply water to the electrolytic cell according to the feedback of a water level detector, and to control the pressure release of the electrolytic cell according to the feedback of a pressure sensor 29, and to control the start of the atomizer 12, and so on.

A side plate sealing element 8 is further disposed between the upper cover 1 and the housing to fix and seal the two parts.

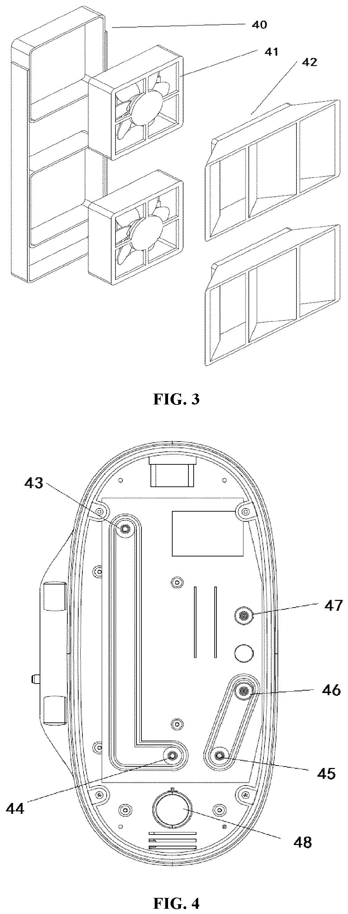

The gas guide plate 13 (as shown in FIG. 4) is provided with a first gas guide groove 15 and a second gas guide groove 14, gas outlets/inlets are arranged at both ends of the gas guide grooves, and the structures of the gas guide grooves are rationally distributed the gas guide plate 13 according to the positions of the gas outlets. The top of the gas guide groove is sealed by a sealing cover 16 of the gas guide groove to form a gas guide chamber for gas to circulate therein.

A cooling plate 17 is further disposed between the water supply tank upper cover 18 and the gas guiding plate 13 for cooling the generated gas.

The water supply tank upper cover 18 is provided with a plurality of outlets/inlets corresponding to the gas outlets/inlets on the gas guide plate 12. The water supply tank upper cover 18 is further provided with a water injection channel 48 connected to a water injection port 6 arranged on the upper cover 1, to supply water to the water supply tank 23. The water injection port 6 is further sealed with a sealing cover 7 of the water injection port. Further, a water pipe under the water supply tank 23 is connected to a drainage opening 52 of the water supply tank, to empty the water stored in the water supply tank 23. In order to monitor the water level of the water supply tank 23, an external sensing-type water level detector for water tanks can also be used for water level monitoring.

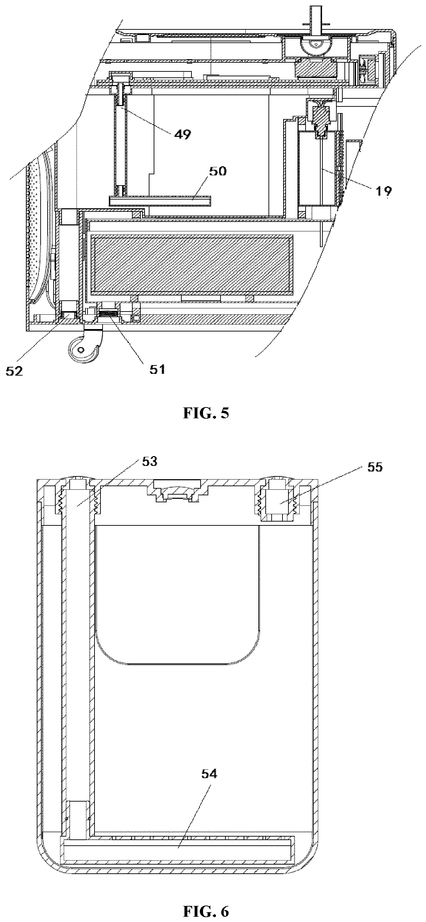

As shown in FIGs. 5 and 6, the water supply tank 23 is further provided with a device inside for generating fine air bubbles, such as an aeration device, to fully wash the gas. Similarly, the secondary water tank 26 is also provided with a device inside for generating fine air bubbles. The secondary water tank 26 is used for secondary cleaning and filtering of the gas. The secondary water tank 26 is installed on one side of the housing through a secondary water tank base 25. The secondary water tank 26 is further used as an observation window for observing the gas washing situation therein.

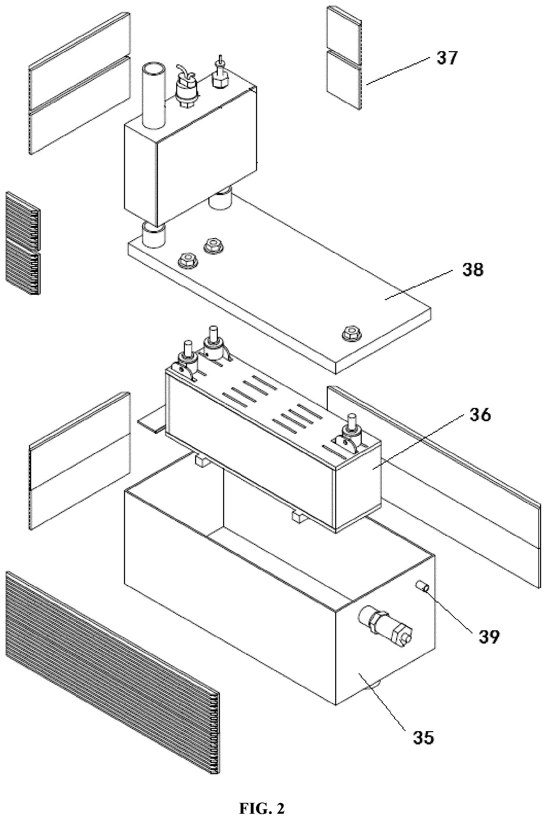

As shown in FIG. 2, the electrolytic cell that the present invention adopts is an electrolytic cell 27 with heat dissipation fins, that is, heat dissipation fins 37 are arranged around an electrolytic tank 35. Specifically, the electrolytic cell is arranged on the bottom cover 33 at the bottom of the housing. The electrolytic cell includes the electrolytic tank 35, and an electrolytic stack 36 and an electrolyte placed inside the electrolytic tank 35. An upper cover 38 of the electrolytic tank 35 is provided with an air outlet pipe 28, a pressure sensor 29, a metal probe insertion port 30 of a liquid level sensor, a safety valve 32 for pressure relief, and an electrolyte injection port 39 connected to a pump, where the electrolyte injection port 39 supplies liquid through the water supply tank 23. The electrolytic cell is further provided with a drainage opening 51 below, to discharge the electrolyte. The electrodes in the electrolytic stack are arranged in the serial-parallel connection mode, and in the case of low voltage input, the energy consumption is relatively low and the use is safer. In order to further fix the electrolytic cell, a fixing bracket 31 is further arranged on the bottom cover 33 to prevent the electrolytic cell from moving.

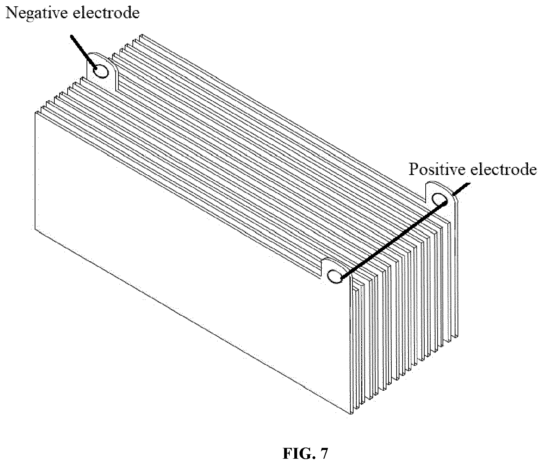

As shown in FIG. 7, for the electrolytic stack, compared with the serial or parallel electrode connection, the serial-parallel electrode connection mode (sharing one negative electrode) features a lower stack input voltage, and enables high gas production and relatively low energy consumption at a safe voltage (<36V). In the following table, the input voltage, input current and power in the serial-parallel mode adopted by the present invention are listed. It can be seen that at the same gas production rate, the input voltage in the serial-parallel mode is lower than that in the serial mode, the current is larger, and the energy consumption is relatively lower; and energy consumption in the serial-parallel mode is lower than that in the parallel mode.

| Single electrode area (cm²) | Number of chambers | Gas production rate (L/min) | Electrode connection mode | Input voltage (V) | Input current (A) | Power (W) |
|---|---|---|---|---|---|---|
| 200 | 16 | 5 | Serial connection | 43.9 | 32 | 1404.8 |
| 200 | 16 | 5 | Parallel connection | 3.4 | 503 | 1710.2 |
| 200 | 16 | 5 | Serial-parallel connection | 22.2 | 63 | 1398.6 |
| 200 | 16 | 3 | Serial connection | 43.2 | 19 | 820.8 |
| 200 | 16 | 3 | Parallel connection | 3.2 | 301 | 963.2 |
| 200 | 16 | 3 | Serial-parallel connection | 21.6 | 38 | 820.8 |
| 200 | 16 | 2 | Serial connection | 43.4 | 13 | 564.2 |
| 200 | 16 | 2 | Parallel connection | 3.1 | 200 | 620 |
| 200 | 16 | 2 | Serial-parallel connection | 22.1 | 25.5 | 563.6 |
| 200 | 16 | 1 | Serial connection | 43.4 | 6.7 | 290.8 |
| 200 | 16 | 1 | Parallel connection | 3.1 | 101 | 313.1 |
| 200 | 16 | 1 | Serial-parallel connection | 22.3 | 13 | 289.9 |

Heat dissipation openings 22 for heat dissipation are arranged on both sides of the housing, and a fan assembly 24 is arranged inside the housing corresponding to the heat dissipation opening (as shown in FIG. 3). The fan assembly 24 includes a fan 41, a fan fixing bracket 40, and a wind direction guide groove 42 arranged at the front end of the fan 41, where the wind direction guide groove 42 faces one side of the electrolytic cell and has a gradually expanded opening used for introducing more air to better dissipate heat of the electrolytic cell.

To better achieve portability, the lower end of the bottom cover 33 is provided with casters 34, and the housing is further provided with a pull rod 21.

The process of using the high-efficiency oxyhydrogen generating device for medical care of the present invention is as follows:

Preparing: open the sealing cover 7 of the water injection port, add an appropriate amount of water to the oxyhydrogen generation device, power on, turn on the power switch (arranged at the bottom of the housing to power the device) on the housing, and then turn on the power button 5 on the upper cover to start the device.

The oxyhydrogen generated by the electrolytic cell is discharged into a first gas outlet 43 of the gas guide plate through the gas outlet pipe 28, and then is introduced into a first gas inlet 44 to enter a second gas inlet 49 through the first gas guide groove 15, and then is fully contacted with the water in the water supply tank 23 through a second gas outlet 50 of the aeration device, to achieve primary cleaning and filtering. The filtered gas is discharged through a third gas outlet 45, enters a fourth gas inlet 53 of the secondary water tank 26 from a third gas inlet 46 after passing through the second gas guide groove 14, then enters the aeration device of the secondary water tank 26, is fully contacted with the water in the secondary water tank for cleaning and filtering after passing through a fourth gas outlet 54, is discharged from a fifth gas outlet 55, then enters a flame arrester through a sixth gas outlet 47, then enters the atomizer 12, and finally is discharged from the atomization port 2.

When the oxyhydrogen generation device works, the metal probe 19 of the water level detector monitors the height of the electrolyte in the electrolytic cell in real time. When the electrolyte height is reduced to a preset threshold, the water supply tank 23 is started to supply water to ensure the normal operation of the device.

The oxyhydrogen produced by the electrolysis of the electrolytic cell of the present invention flows into the water supply tank 23 through the gas outlet pipe 28 and the first gas guide groove 15 on the gas guide plate 13 in the upper cover 18 of the water supply tank for the purpose of primary cleaning, and then enters the secondary water tank 26 after passing through the second gas guide groove 14 on the gas guide plate 13 for secondary cleaning. The cleaned gas can be optionally discharged from the atomized gas circulation part through the atomizer 12 or directly discharged for use by users.

Finally, it should be noted that the above embodiments are only used to illustrate the technical solutions of the present invention, but not to limit them; although the present invention has been described in detail with reference to the foregoing embodiments, those ordinarily skilled in the art should understand that: the technical solutions described in the foregoing embodiments can still be modified, or some or all of the technical features thereof can be equivalently replaced; and these modifications or replacements do not make the essence of the corresponding technical solutions deviate from the scope of the technical solutions of the embodiments of the present invention.

## Claims

1. A high-efficiency oxyhydrogen generation device for medical care, comprising:
a housing configured to hold an electrolytic cell, a water supply tank, and a secondary water tank;
an upper cover fastened to an upper part of the housing and provided with a gas circulation part, a supply part for supplying water to the water supply tank and a control panel for controlling the operation of an electrolyzing water hydrogen-oxygen generator;
a bottom cover fastened to an bottom of the housing;
wherein,
the water supply tank is provided with a water supply tank upper cover, an accommodating space is disposed inside the water supply tank upper cover, the upper cover is sleeved on the outer side of the water supply tank upper cover and is fastened to the housing, and an air guide plate, a circuit board, an atomizer, and a fixing plate for fixing the circuit board are disposed in the accommodating space;
the electrolytic cell is disposed on the bottom cover at the bottom of the housing, and is connected to the water supply tank thereabove through a pipeline, the oxyhydrogen generated by the electrolysis of the electrolytic cell passes through a gas outlet pipe and the air guide plate in the water supply tank upper cover to enter the water supply tank for primary cleaning, and then enters the secondary water tank through the air guide plate for secondary cleaning, and the cleaned gas is discharged from the gas circulation part.

2. The high-efficiency oxyhydrogen generation device for medical care according to claim 1, wherein the electrolytic cell is an electrolytic cell with heat dissipation fins, and comprises an electrolytic tank, and an electrolytic stack and an electrolyte placed inside the electrolytic tank, and the electrodes in the electrolytic stack are disposed in a serial-parallel connection mode.

3. The high-efficiency oxyhydrogen generation device for medical care according to claim 2, wherein the electrolytic tank is provided with a gas outlet pipe, a pressure sensor, a metal probe insertion port of a liquid level sensor, a safety valve, and a water injection port connected to a pump, wherein the water injection port is connected to the water supply tank through a pipeline.

4. The high-efficiency oxyhydrogen generation device for medical care according to claim 2, wherein the heat dissipation fins are disposed around the electrolytic tank, heat dissipation openings for heat dissipation are disposed on both sides of the housing, and a fan assembly is disposed inside the housing corresponding to each the heat dissipation opening.

5. The high-efficiency oxyhydrogen generation device for medical care according to claim 1, wherein two gas guide grooves are disposed on the air guide plate, a gas outlet and a gas inlet are disposed at both ends of each the gas guide groove, and a gas guide groove sealing cover is disposed on each the gas guide groove.

6. The high-efficiency oxyhydrogen generation device for medical care according to claim 1, wherein each of the water supply tank and the secondary water tank is further provided with a device for generating fine bubbles.

7. The high-efficiency oxyhydrogen generation device for medical care according to claim 1, wherein the secondary water tank is installed on one side of the housing through a secondary water tank base, and the secondary water tank is further used as an observation window for observing the gas washing situation therein.

8. The high-efficiency oxyhydrogen generation device for medical care according to claim 1, wherein an atomizer is fixed on the fixing plate, and the gas after secondary washing is discharged from the gas circulation part after optionally passing through the atomizer.

9. The high-efficiency oxyhydrogen generation device for medical care according to claim 4, wherein the fan assembly comprises a fan, a fan fixing bracket, and a wind direction guide groove disposed at the front end of the fan.

10. A method for using the high-efficiency oxyhydrogen generation device for medical care according to any one of claims 1 to 9, wherein the oxyhydrogen generation device is started after adding an appropriate amount of water; oxyhydrogen generated by the electrolytic cell is discharged into an gas guide channel on the air guide plate through the gas outlet pipe and enters the water supply tank for primary cleaning, and then enters the secondary water tank through the gas guide plate for secondary cleaning, and the cleaned gas is directly discharged from the gas circulation part or discharged after passing through the atomizer.
